# EUROPEAN PATENT APPLICATION

(11) **EP 1 852 097 A2**
(43) Date of publication of application: **07.11.2007**
(21) Application number: 07008620.2
(22) Date of filing: 27.04.2007
(51) Int. Cl.: A61F 13/02

(54) **An adhesive bandage**

(30) Priority: 03.05.2006 BR PI0601479
(71) Applicant: Johnson & Johnson Industrial Ltda., 12237-350 Sao José dos Campos SP (BR)
(72) Inventor: Da Silva Macedo, Carlos Jr., Sao José dos Campos Sao Paulo (BR)
(74) Representative: Metten, Karl-Heinz

(57) **Abstract**

The present invention relates to a multilayer adhesive bandage comprising:
- at least one first support layer (2);
- at least one first adhesive absorbent layer (3), deposited onto the first support layer (2); and
- at least one first discontinuous wound-contact component (4), substantially free from hydrocolloids, for substantially covering a wound area, said wound area having a surface on which the first adhesive absorbent layer (3) comes into substantial contact with said wound surface through the first discontinuous wound-contact component (4).

## Description

The present invention relates to an adhesive bandage to be applied specially onto the skin, particularly an adhesive bandage having great flexibility of use after having been applied onto the skin without, however, causing any damage to the injury upon removal. The invention further relates to a process for manufacturing this adhesive bandage.

### Description of the Prior Art

Adhesive bandages intended for use on the skin, protecting the skin from injuries or the wound (s) from dirt and guaranteeing the efficacy of medicaments applied topically, comprise essentially at least one film made of a liquid-impermeable material and that also prevents contamination of the wound.

Conventionally, at least one pad comprising at least one absorbent material and at least one adhesive segment is associated to this film, in order to enable fixation of the adhesive bandage to the user's skin. Preferably, the adhesive element is positioned on the adhesive bandage in such a manner that the region intended to be in contact with the would (the pad) does not have adhesive, since the presence thereof could entail maceration at the wound upon removal or replacement of the adhesive bandage.

In order to accelerate the healing of the wound and enable skin respiration, the film is permeable to gas and may further contain a plurality of through bores.

With a view to increase more and more the efficacy and the comfort provided by the adhesive bandage, a number of improvements in this extremely efficient basic concept were developed, as for instance, the improvement of the materials used (more flexible, comfortable, inexpensive, with greater capacity of absorbing fluids, etc.) or the form of alterations in the coloration of the adhesive bandage (transparent, colored, provided with children's characters, etc.).

A first type of adhesive bandage is disclosed and defined in US Pat. 5,465,737 (Patel), and is essentially constituted by two layers, between which an absorbent element is positioned to receive the bodily fluids resulting from the healing process of a wound.

A first layer, permeable to liquid, enables the positioning of the adhesive bandage over the user's skin, while the second layer, impermeable to liquid but permeable to gas, acts as a barrier against the exit of bodily exudates and still prevents dirt and pathogenic agents such as bacteria from reaching the wound. The first layer further has an adhesive element applied thereto, which enables one to fix the adhesive bandage to the user's skin. The adhesive used in this absorbent adhesive bandage may preferably comprise hydrocolloids.

The absorbent element has smaller dimensions if compared with the two end layers and is positioned in a centralized manner with respect to them, so as to leave a sufficient area of the first layer impregnated with adhesive for fixing the adhesive bandage to the skin.

The absorbent adhesive may have several preferred embodiments. However, it comprises essentially at least one portion composed of at least one low-density absorbent element and one portion composed of at lest one high-density absorbent element contiguous to each other.

Another type of adhesive bandage is disclosed and defined in US Pat. 4,561,435 (Mcknight et al) and comprises an absorbent pad, a film that is impermeable to liquid and permeable to gas, positioned on one of the surfaces of this pad, adjacent to which a layer of adhesive film or fabric is positioned, this film having a window in the region of association with the pad, especially in the central region delimited by the pad. The liquid-impermeable film prevents dirt and pathogenic agents from reaching the wound.

The adhesive bandage further comprises at least one liquid-permeable anti-adherent film, positioned on the other surface of the pad, allowing the bodily exudates from the wound to reach the pad, at the same time preventing it from sticking to the healing wound, which would be extremely undesirable at the time of removing or replacing the adherent bandage.

A third type of adhesive bandage is disclosed and defined in US Pat. 5,681,579 (Freeman), which has a main layer composed of a hydrocolloid polymer composite or a polymer covered with hydrocolloid and an opposite impermeable layer. Further, an adhesive is provided on the main layer to enable fixation thereof to the user's skin and an absorbent layer is provided between the permeable layer with hydrocolloid and the impermeable layer.

Finally, a fourth type of adhesive bandage is defined and claimed in document EP 0617938, which comprises a support layer composed of a polymer containing hydrocolloid, positioned directly over the user's skin, thanks to an adhesive applied to it, as well as an impermeable layer positioned over the support layer, preventing contact of dirt and pathogenic agents with the wound.

All the adhesive bandages mentioned above, in spite of presenting configurative variations as far as the particular constitution is concerned and in the materials used, have a common disadvantage, namely the big thickness resulting from the fact that they have an impermeable layer, an adsorbent element and a permeable layer facing the wound. This big thickness, especially if it is located in articulated regions of the body (fingers, knees, wrists, etc.) cause the adhesive bandage to deform rapidly, possibly detaching from the skin, or allowing the entry of dirt. Thus, the useful life of the adhesive bandage is reduced, since due to the detachment it needs to be replaced before the absorption capacity of the pad is completely exhausted.

With a view to eliminate these drawbacks, one developed adhesive bandages composed of a single film that is impermeable to liquid and permeable to gas, to which a layer of adhesive containing hydrocolloid was added. With that the adhesive bandage had an extremely reduced thickness and a capacity of absorbing liquids that, if not equivalent to that of an adhesive bandage provided with very thick pads, at least it was sufficient for use on minor cuts/injuries, which are the large majority of the cases of superficial injuries.

The flexibility resulting from its small thickness caused the adhesive bandage to remain adhered to the skin for along time, even if the wound were located in regions of body articulations, so that the replacement thereof would only be necessary after saturation of the absorption capacity of the hydrocolloid. With that, the amount of adhesive bandages used until a skin injury has been recovered has decreased, whereby the expenditures of the user with the product are reduced.

As an additional advantage, it was possible to configure the film so as to mask as much as possible the existence of the adhesive bandage, or to make it transparent, so that it would be hardly noticed when in use.

The great disadvantage of this type of adhesive bandage was its strong adhesion to the wound, which cased new injury at the time of removing the adhesive bandage for replacement. This was due to the impossibility of controlling the rate of adhesion of the adhesive bandage to the wound, since it was composed of a single film provided with adhesive containing hydrocolloid. Besides, it did not provide any masking of the wound (that is to say, the capacity of hiding the existence / appearance thereof), which causes discomfort to the user.

Additionally, one had not yet developed any adhesive bandage having all the advantages already commented on and that could have utilization other than as a dressing for wounds, such as a protective utilization (to prevent injuries) or as an absorbent product (breasts protection or sanitary napkins).

### Objectives of the Invention

The present invention has the objective of providing an adhesive bandage for use on skin wounds, as well as for protecting the skin in high-demand regions or as absorbents, which has the antagonistic characteristics of high capacity of peripheral adhesion and possibility of controlling adhesiveness in its central region, thus bringing about advantages such as periodic replacement thereof so as to provide a comfortable change to the user, rapid healing, without re-injuring the wound and absence of undesirable discoloration and as much discretion as possible.

Also, the present invention has the objective of providing a process for manufacturing the adhesive bandage aimed at.

### Brief Description of the Invention

The objectives of the present invention are achieved by means of a multilayer adhesive bandage that comprises:
- at least one first support layer;
- at least one first adhesive absorbent layer, deposited on the first support layer; and
- at least one first discontinuous wound-contact component for substantially covering a wound area, said wound area having a surface on which the first adhesive absorbent layer substantially comes into contact with said wound surface through the first discontinuous wound-contact component.

Also, the objectives of the present invention are achieved by means of a multilayer adhesive bandage for healing injuries that comprises:
- at least one first support layer;
- at least one first adhesive absorbent layer, deposited on the first support layer; and
- at least one first discontinuous wound-contact component, substantially free from hydrocolloids, for substantially covering a wound area, said wound area having a surface, on which the first adhesive absorbent layer substantially comes into contact with said wound surface through the first discontinuous wound-contact component.

Finally, the objectives of the present invention are achieved by means of a multilayer adhesive bandage for healing injuries, which comprises at least one first support layer, at least one first adhesive absorbent layer, deposited on the first support layer, at least one first discontinuous wound-contact component, substantially free from hydrocolloids, for substantially covering a wound area, the first discontinuous wound-contact component being an integral part of said first adhesive absorbent layer.

### Brief Description of the Drawings

The present invention will now be described in greater detail with reference to an embodiment represented in the drawings. The figure show:
- Figure 1 is a perspective schematic view of a first adhesive bandage of the prior art;
- Figure 2 corresponds to two sectional schematic views of the adhesive bandage illustrated in figure 1;
- Figure 3 is a perspective schematic view of a second adhesive bandage of the prior art;
- Figure 4 corresponds to a sectional schematic view of the adhesive bandage illustrated in figure 3;
- Figure 5 is a perspective schematic view of a first preferred embodiment of the adhesive bandage of the present invention;
- Figure 6 corresponds to a sectional schematic view of the adhesive bandage illustrated in figure 5;
- Figure 7 is a perspective schematic view of a second preferred embodiment of the adhesive bandage of the present invention;
- Figure 8 corresponds to a sectional schematic view of the adhesive bandage illustrated in figure 7;

### Detailed Description of the Figures

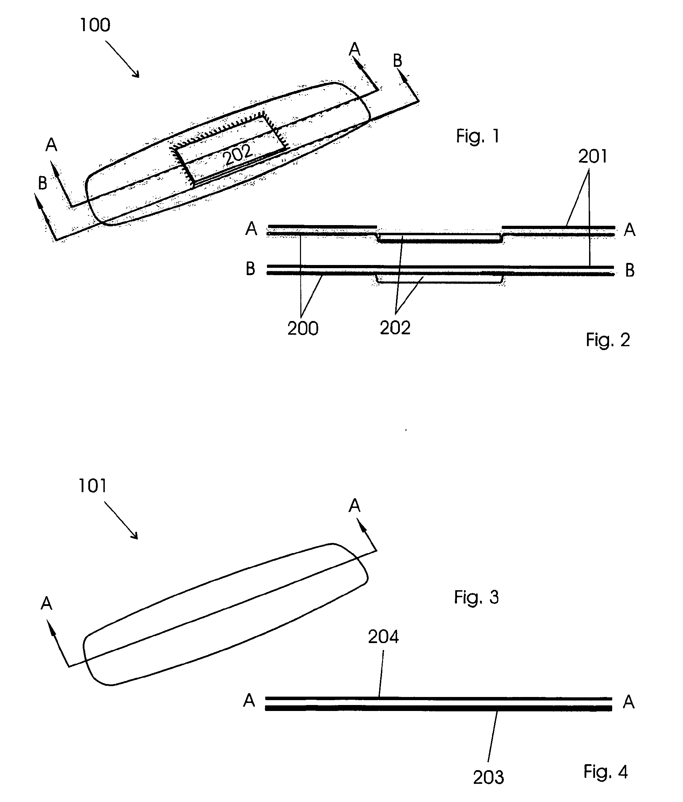

Figures 1 to 4 illustrate adhesive bandages of the prior art, already known before.

In figures 1 and 2, one can see a first adhesive bandage 100, known from the art, comprising a main impermeable plastic film 200, to which a very thick absorbent pad 202 is associated, preferably by means of glue. The sectional views A-A and B-B enable one to see that the impermeable film 200 is impregnated with adhesive 201 throughout its length, with the exception of the region dominated by the pad 202, since it must not adhere to the wound in any way, under pain of causing additional injuries when the adhesive bandage 100 is removed fro the skin.

This adhesive bandage 100 is already known, although it has good capacity of absorbing bodily exudates resulting from the healing of the wound due to the very thick absorbent pad 202, has little flexibility and, for this very reason, ends up detaching from the skin long before the pad 202 becomes saturated with liquid products, thus requiring early replacement. Thus, it may not be economically interesting to manufacture an adhesive bandage 100 configured in this manner, wherein the pad has a really high capacity of absorbing exudates, since the adhesive bandage will very probably become unfit for use long before its saturation.

Moreover, this type of bandage is not efficient in use as protective for the skin or even as protective for one's breasts or as sanitary napkins.

A second type of adhesive bandage 101 too is already disclosed in figures 3 and 4 and comprises a single film 203 that is impermeable to liquids and permeable to gas, to which a layer of adhesive 204 containing hydrocolloid is added. The resulting adhesive bandage has a considerably reduced thickness with respect to the adhesive bandage 100 of figures 1 and 2 and a capacity of absorbing liquids that, even though it is not equivalent to that presented by the former, is still optimal for use on minor cuts/injuries, which are the large majority of the cases of superficial injuries, or for use as protective against injuries, as for instance, in the case of shin-guards used by sportsmen.

Due to the characteristics of the component materials and to its reduced thickness, the adhesive bandage 101 is highly flexible and so it remains adhered to the skin for a long time, even if positioned in regions of articulations of the body. In this way, it is possible that it will remain in use until saturation of the hydrocolloid, and only then will it be replaced. Optionally, this adhesive bandage 101 is configured so as to mask as much as possible its existence (exhibiting a finish in the same color as the skin, opaque translucent, etc.).

A first variation of this adhesive bandage 101 comprises a single adhesive layer and hydrocolloid 204, called a mono-component, since it is the only one existing in this case. In this way, one provides an adhesive bandage that exhibits the capacity of even and considerable adherence over its whole area, including the region to be positioned over the wound, which is undesired, since this may cause re-injury.

A second variation of the adhesive bandage 101 comprises a double layer of adhesive and hydrocolloid 204, for which reason it is called bi-component. With this, one can apply a first layer composed of much hydrocolloid and litter adhesive over the film area 203 and apply onto it a second layer provided with much adhesive, but without applying it to the region that will come into contact with the wound. In this way, the strong adhesion and the capacity of the adhesive layer 101 to absorbing liquids are ensured, without it having the drawback of sticking to the wound.

Evidently, the adhesive bandage 101 having the bi-component adhesive layer and hydrocolloid has manufacture cost and sale cost considerably higher than those of the adhesive bandage having a mono-component layer, which greatly limits its market penetration.

However, regardless of its configurative variation, a great disadvantage of this type of adhesive bandage is the impossibility of controlling its adhesiveness rate onto the wound, since it comprises only one film, to which a hydrocolloid is applied.

The adhesiveness corresponds to the sticking capacity of a bandage surface (or any other product having adherent adhesives) to adhere to the user's skin or to any other surface.

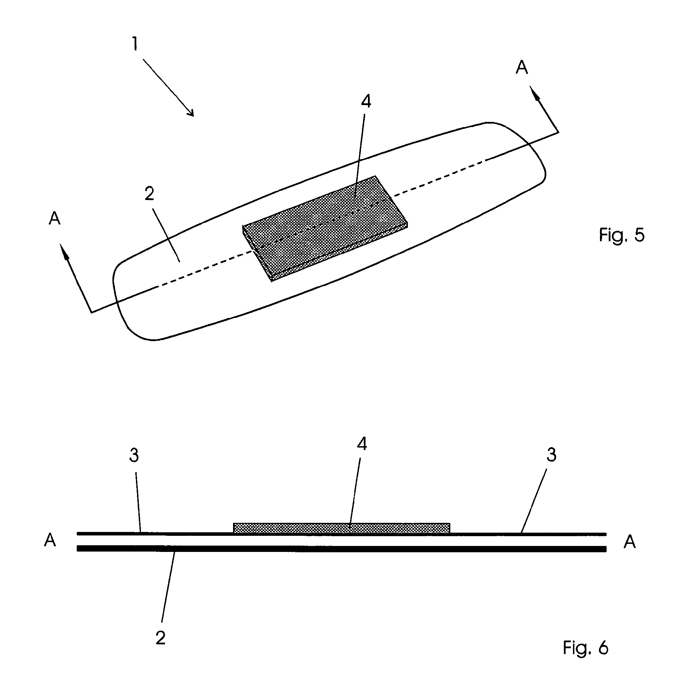

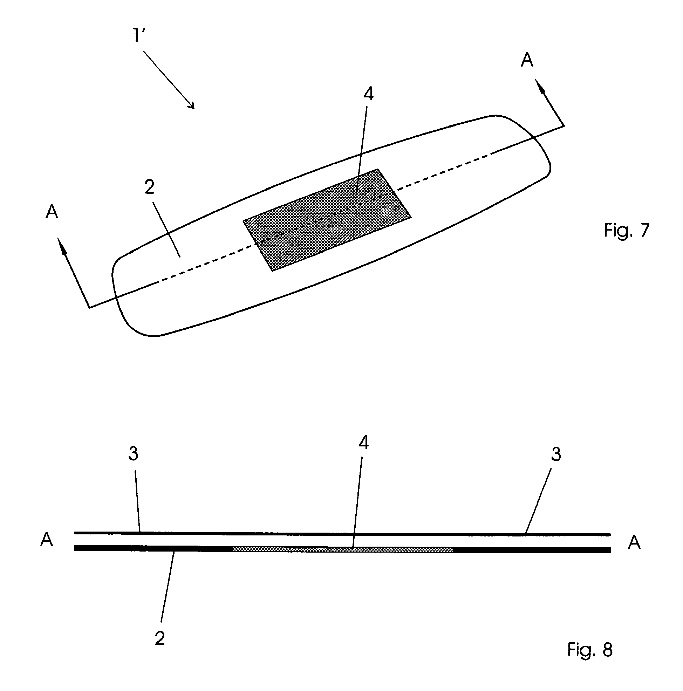

Figures 5 to 8 illustrate two preferred embodiments of the adhesive bandage of the present invention. More particularly, a first preferred embodiment 1 is show in figures 5 and 6, and a second preferred embodiment 1' is shown in figure 7 and 8.

Regardless of its preferred embodiment, the adhesive bandage 1, 1' comprises at least one first support layer 2, to which at least one first adhesive absorbent layer 3 is associated.

The first support layer 2 may have various shapes, as desired (rectangular, circular, oblong, etc.) and its shape will be that presented by the adhesive bandage 1, that is to say, the shape of the adhesive bandage 1, 1' is defined by the first support layer 2. in this way, the composition of the first support layer 2 may vary, but it is preferably made from a polyolefin, polyurethane polymer, polyethylene, vinyl polyethylene acetate, polyurethane foam film, and may further be made from a textile non-woven fabric, rubber, etc.

By preference, the first support layer 2 exhibits viscosity of about 500 to 500,000 centipoises at temperatures lower than 190°C, more preferably, the first support layer 2 exhibits viscosity of about 1,000 to 30,000 centipoises at temperatures lower than 190C, and still more preferably the first support layer 2 exhibits viscosity of about 3,000 to 15,000 centipoises at temperatures lower than 190°C.

Further preferably, the first support layer 2 is impermeable to liquid but permeable to gas, which allows the wound and the skin to which the adhesive bandage 1, 1' is adhered to breath. For this, the polymer has pores of such a size that will allow only the passage of gases, which are molecules of extremely small size. Finally, one can conceive a layer 2 that is perforated for more ventilation of the skin. Alternatively, the first support layer 2 may still be totally impermeable to gases, when necessary.

The first adhesive absorbent layer 3 comprises an adhesive, so that it will be able to stick to the user's skin.

The first adhesive absorbent layer 3 may further contain, by preference, discrete hydrocolloids. By discrete elements one understands different particles of reduced size in an adhesive medium as taught in documents US 5,643,187 and US 6,558,792, incorporated herein by reference. The hydrocolloid element used may be any substance that has a good performance in this utilization, as for example, sodium carboxymethylcellulose, pectin, xanthan gum, polysaccharides, sodium or calcium alginates, chitosan, seaweed extract (cageenan), polyaspartic acid, polyglutamic acid, hyaluronic acid or salts and derivatives thereof, among others.

Hydrocolloids, just as sodium carboxymethylcellulose and pectin, among others, are agents that form gels as soon as they come into contact with the bodily fluids from the wound. When used in adhesive bandages, these hydrocolloids are combined with elastomers and/or adhesives (the composition of which will be mentioned later). Preferably, the adhesive bandage 1, 1' should guarantee a humid environment but without saturation, cicatrisation, which is a situation suitable for acceleration of the healing,

Pectin is a complex-structure polysaccharide, extracted from vegetable species (as for example, peels from citric fruits or apple pulp), which has a highly hydrophilic structure and, as a result, associate easily with the water molecules of the bodily fluids from the wound, forming a viscous gel on the injury bed. Its chemical similarity with alginates causes the physical properties of absorption and gel formation to resemble each other.

Carboxymethylcellulose, in turn, is a cellulose derivative, formed by reaction of cellulose with alkalis (such as, for example, sodium, potassium, calcium, etc., hydroxide). It is the nature of combined alkali that imparts the ionic characteristic of carboxymethylcellulose (when sodium hydroxide is used, sodium carboxymethylcellulose is formed). Just as in the case of pectin, carboxymethylcellulose dissolves rapidly in the water coming from the liquids that emanate from the wound, forming a gel on the wound with controlled viscosity.

As an additional advantage of the use of hydrocolloids, it should be noted that both pectin and carboxymethylcellulose form a gel with acidic characteristics (pH of about 4), functioning as an bactericidal agent.

Before the use of the adhesive bandage 1,1', hydrocolloid is substantially inert to water vapor; but, as soon as the gelling process begins, the adhesive bandage 1, 1' becomes progressively more permeable. The gelling process continues, provided that the injury continues to exude bodily fluids, until the whole hydrocolloid is used up, at which time saturation of the adhesive bandage 1, 1' is reached, and it should be replaced.

In the same way, the adhesive element used may be any one, as for example pressure acrylic adhesives, among others. Additionally, such an adhesive may contain a resin for increasing adhesion, a cohesion increasing agent, an absorption agent (preferably a polyacrylate superabsorbent, a polyacrylate salt superabsorbent or a mixture thereof), a plasticizer and optionally a pigment. The first adhesive absorbent layer 3 may further be configured in discontinuous patterns, arranged in lines, screen, spray or any other which a person skilled in the art understands as discontinuous, composed by an elastomeric base.

Over the first adhesive absorbent layer 3 one positions at least one first discontinuous wound-contact component 4, which substantially covers the wound area and allows the first adhesive absorbent layer 3 to come into substantially contact with the wound surface.

Particularly, the first discontinuous wound-contact component 4 is configures as a screen having openings, which in a first preferred embodiment 1 of the adhesive (see figures 5, 6) is positioned substantially centralized over the first adhesive absorbent layer 3 and remains adhered in that position exactly by action of the first adhesive absorbent layer 3. However, it is clear the first discontinuous wound-contact component 4 may assume other shapes than screen, as for instance a perforated film, a fabric, a non-woven fabric, a perforated non-woven fabric, a gaze, a wax film, a film of a hydrophobic material or a powder film, a powder, wax points, a screen of a polymeric material, a fiber grid, etc. Whatever the configuration of the first discontinuous wound-contact component 4, it is not continuous, that is to say, it has empty spaces/opening that, as mentioned above, enable it to allow the first adhesive absorbent layer 3 to come into substantial contact with the wound surface.

Exemplifying, the first discontinuous wound-contact component 4 has discontinuities selected from pores, slots, perforations, cracks, grooves, openings, holes or the like, or mixtures thereof.

Further detailing the first discontinuous wound-contact component 4, one observes that it preferably has an open area referring to the sum of the discontinuities (or a plurality of discontinuities) of about 10% to 90%, more preferably having an open area of about 25% to 60% and still more preferably of about 30 to 40%.Further, one considers to be ideal a pore area of about 0.001 to 10mm², preferably from 0.1 to 1mm² and more preferably of about 0.1 to 0.5mm².

A second preferred embodiment 1' of the adhesive bandage (see figures 7, 8), in turn, has the screen 4 as an integral part of the first support layer 2, this constitution being preferably but not compulsorily obtained by means of joint extrusion with the layer 2.

The positioning of the first discontinuous wound-contact component 4 over the first support layer 2 brings, as advantages, lower manufacture cost of the adhesive bandage 1, but, in contrast, there is an increase in its total thickness. On the other hand, the use of the first discontinuous wound-contact component 4 as an integral part of the first support layer 2 confers to the adhesive bandage 1' a larger thickness without alteration in the absorption capacity, but has, as an advantage, a higher manufacture cost.

Whatever the embodiment of the adhesive bandage 1, 1', however, one should note that the first discontinuous wound-contact component 4 is made preferably from a polymeric material. But the possibility of making it from a natural fibrous material such as gaze or any other textile material is not ruled out, and it is preferably impregnated with bactericidal products or various active principles (for example, healing products) in order to obtain more clinical / operational efficiency of the adhesive bandage 1, 1'. This situation occurs because it is the first discontinuous wound-contact component 4 that remains in direct contact with the wound, enabling these products to actuate. For the same reason, it should have anti-sticking properties, in order to have negligible surface adherence and not to hurt the injuries further, when the adhesive bandage is removed and/or replaced (re-injury).

The existence of the first continuous wound-contact component 4 is a grant advantage of the adhesive bandage 1, 1' over the adhesive bandage illustrated in figures 3 and 4, since it enables one to manage the adhesiveness of the adhesive bandage 1, 1' onto the wound, beside comprising preferably impregnated products. In other words, the first discontinuous wound-contact component 4 has the capacity of managing the adhesiveness of the adhesive absorbent layer 3. One should also note that the adhesiveness or adhesion capacity of the adhesive bandage may be determined according to the amount and/or area and/or shape and/or arrangement of the openings and/or thickness of the first discontinuous wound-contact component 4.

A larger open area of the first discontinuous wound-contact component 4 associated to a larger number of openings or to the existence of openings with a large area (which implies a larger area wound-contact area) provides adhesion of the adhesive bandage to the skin, since the increase in the amount and/or area and/or shape and/or arrangement of the openings of the first discontinuous wound-contact component 4 brings about a larger empty space to be filled up. These openings serve as real channels, which allow the adhesive containing hydrocolloids to pass, thus enabling one to anchor the bandage onto the skin or wound and favoring close contact of the absorbent hydrocolloid material with the wound. It should be further reminded that it is the first discontinuous wound-contact component 4 that comes into contact with the skin or injured site. So, the larger the amount of adhesive and hydrocolloid existing therein the greater adhesiveness of the corresponding bandage.

Analogously, a corresponding reduction in the wound-contact area (due to the reduction of the screen and/or reduction of the area and/or amount of the openings) will cause a drop in the adhesion of the adhesive bandage to the skin. When the openings of the first discontinuous wound-contact component provide a reduced total passage area, there is a lesser amount of adhesive in contact with the skin or wound, thus reducing the adhesion of the adhesive bandage thereto and minimizing the anchorage thereof, which causes, at the time of replacing the bandage, a less aggressive removal, without any injury to the skin or removal of tissue reconstituted by cicatrisation.

By virtue of the characteristics explained above, the handling of the area of the first discontinuous wound-contact component 4 and of the amount and area of its openings enables one to manufacture adhesive bandages 1, 1' for the most varied uses.

One can manufacture an adhesive bandage 1, 1,' having high adhesiveness to the skin for protection against injuries (in order to prevent injuries/bruise), in order to prevent damage to the skin in high-requirement regions, such as the heels or elbows. This type of protective bandage is very suitable for use by sportsmen, for instance.

One may also manufacture a adhesive bandage 1, 1'for use as a dressing on bruises/wounds, which has high adhesiveness to the bruise in the peripheral region, combined with less adhesiveness in the central region and healing capacity combined with comfort of the user at the time of replacement.

By preference, the first discontinuous wound-contact component 4 defines a wound-contact region 18, exhibiting a first adhesiveness, and the first adhesive layer 3 not covered by the first discontinuous wound-contact component 4 defines a skin-contact region 19, exhibiting a second adhesiveness, such that the first adhesiveness will be 10% lower than the second adhesiveness.

Therefore, the first adhesiveness value is preferably of at least 36N /cm2 and the second adhesiveness value is of at least 40 N/cm2. More preferably, the first adhesiveness value is of at least 25 N/cm2 and the second adhesiveness value is of at least 36 N/cm2. Still more preferably, the first adhesiveness value is preferably of at least 10 N/cm2 and the second adhesiveness value is of at least 25 N/cm2. Other preferred first adhesiveness values are 12 N/cm2, 8 N/cm2 and 4 N/cm2, and second adhesiveness values are 15 N/cm2, 10/cm2 and 8 N/cm2, respectively.

Still preferably, other first adhesiveness values may be, at the most, 10 N/cm2, preferably 6 N/cm2 and more preferably 1 N/cm2.

In addition to the above examples, one can use the adhesive bandage 1, 1' of the present invention as a breast protector and even as a sanitary napkin, by just manipulating the characteristics of the first discontinuous wound-contact component 4 (total area, amount, positioning and opening area), so that its performance will be adequate as far as the fluid absorption capacity is concerned.

It is further possible for the bandage to be one of the components of a breast protector or an absorbent composes of still other elements and layers, so as to achieve the desired absorption.

Therefore, considering a fixed adhesive volume, an adhesive bandage 1, 1' whose first discontinuous wound-contact component 4 exhibits a high sum of the areas of the openings will exhibit high adhesiveness, since the area of adhesive exposed will be equal to the sum of the areas, but, in contrast, will exhibit high wound masking (that is to say, capacity of hiding the existence/appearance thereof). In contrast, an adhesive bandage 1,1' whose first discontinuous wound-contact component 4 exhibits a reduced sum of the areas of openings will exhibit low adhesion to the wound and low masking.

In other words, one can manage the adhesiveness of the adhesive bandage to the wound by manipulating the characteristics of the first discontinuous wound-contact component 4, which is the element that remains directly in contact with the wound.

For using the adhesive bandage on a wound that releases a large amount of liquids, an adhesive bandage 1, 1' having a first discontinuous wound-contact component 4 provided with a large opening area will be preferred, in order to prevent the wound from being excessively wet, thus guaranteeing the closeness between the wound and the hydrocolloid material. On the other hand, in the case where the wound releases a small amount of liquids, the adhesive bandage 1,1' with the first discontinuous wound-contact component 4 provided with a reduced opening area is preferred, since it maintains litter wetness of the wound and saturates more slowly.

Preferably but not restrictively, the first discontinuous wound-contact component 4 is located substantially centralized with respect to the adhesive bandage 1,1' and has a contact periphery surrounding the wound. Still preferably, the first discontinuous wound-contact component 4 has a first surface area and the adhesive bandage 1,1' has a second surface area, such that the first surface area is at least 5% smaller than the second surface area, preferably 10% smaller, 30% smaller and more preferably 50% smaller. This difference in areas enables the first discontinuous wound-contact component 4 to expose the first adhesive absorbent layer 3 to the skin surrounding the wound, permitting anchorage of the bandage 1,1' to the skin of the user and guaranteeing higher adhesiveness in this region, in comparison with the adhesiveness of the first adhesive absorbent layer 3 to the wound, which is reduced due to the first discontinuous wound-contact component 4, as already discussed before. Moreover, the exposed area of the first adhesive absorbent layer 3 is preferably continuous in order to achieve complete sealing around the wound, thus guaranteeing that no dirt will penetrate and contaminate the wound.

Further with regard to the employ of thee adhesive bandage 1,1', it should be noted that there are three concepts to be considered, namely: (i) the MVTR (Moist Vapor Transmission Rate), (ii) absorption and (iii) the "fluid handling".

The MVTR (i) is the permeability of the adhesive bandage 1,1' to the passage of water molecules from the contact surface of the skin to the outer atmosphere, under controlled conditions of moisture and temperature,; the absorption (ii) is the amount of liquid that the adhesive bandage 1,1' is capable of absorbing and, finally, the "fluid handling" (iii) is given by the capacity of the adhesive bandage to distribute liquid in order to obtain better utilization thereof.

Another already cited characteristic of the adhesive bandage 1,1' of the present invention is that, if desired, both the first support layer 2 and the first adhesive absorbent layer 3 and even the first discontinuous wound-contact component 4 can exhibit variation in coloration, for instance, for masking the wound or bruise, or for any other purpose.

The process of manufacturing the adhesive bandage 1,1' of the present invention usually comprises essentially the following steps:
Step (i): obtaining a first support layer 2 as defined before;
Step (ii): obtaining at least one first adhesive absorbent layer 3;
Step (iii): associating the component of the first adhesive absorbent layer 3 to the first support layer 2.

Evidently, the first support layer 2 and the first adhesive absorbent layer 3 can be obtained by any methods available at present, as for instance, extrusion for obtaining the layer 2. In the same way, the obtainment of the first adhesive absorbent layer 3 comprising hydrocolloid or a mixture of hydrocolloid and adhesive can be made in any known manner. One further reminds that the steps (i) and (ii) can be carried out in the mentioned order (first the step (i)), then the step (ii), in the inverted order or event concomitantly.

Alternatively, the step (i) comprises concomitantly extruding the first support layer 2 and at least one first discontinuous wound-contact component 4 (co-extrusion), which is what happens in the production of the of the second preferred embodiment 1' of the adhesive bandage, or still another necessary or desirable procedure.

On the other hand, in the case of manufacturing the first preferred embodiment 1 of the adhesive bandage, the process further comprises a step (v) of positioning at least one first discontinuous wound-contact component 4 in the first support layer 2 over the first adhesive absorbent layer 3, by preference substantially centralized.

Two preferred embodiments having been described, it should be understood that the scope of the present invention embraces other possible variations, being limited only by the contents of the accompanying claims, which includes the possible equivalents.

## Claims

1. A multilayer adhesive bandage, **characterized by** comprising:
- at least one first support layer (2);
- at least one first adhesive absorbent layer (3), deposited onto the first support layer (2); and
- at least one first discontinuous wound-contact component (4) for covering substantially a wound area, said wound area having a surface on which the first adhesive absorbent layer (3) substantially comes into contact with the wound surface through the first discontinuous wound-contact component (4).

2. A multilayer adhesive bandage for wound care according to claim 1, **characterized in that** the first discontinuous wound-contact component (4) defines a first wound-contact region (18) having a first adhesiveness value and a second skin-contact region (19) outwardly of the first discontinuous wound-contact component (4) having a second adhesiveness value, the first adhesiveness value being lower that the second adhesiveness value, more preferably the first adhesiveness value being 10% lower than the second adhesiveness value.

3. A multilayer adhesive bandage for wound care according to claim 1 or 2, **characterized in that** the first discontinuous wound-contact component (4) has an open area of about 10% to about 90% of the total area of the first discontinuous wound-contact component (4), for allowing the first adhesive absorbent layer (3) to come substantially into contact with the wound surface.

4. A multilayer adhesive bandage for wound care according to claim 3, **characterized in that** the first discontinued wound-contact component (4) has an open area of 10% to 70% of the total area of the first discontinuous wound-contact component (4), for allowing the first adhesive absorbent layer (3) to come substantially in contact with the wound surface.

5. A multilayer adhesive bandage for wound care according to claim 4, **characterized in that** the first discontinuous wound-contact component (4) has a total area and an open area substantially comprising about 25% to about 60% of the total area of the first discontinuous wound-contact (4) component, for allowing the first adhesive absorbent layer (3) to come into substantial contact with the wound surface.

6. A multilayer adhesive bandage for wound care according to claim 5, **characterized in that** the first discontinuous wound-contact component (4) has an open area preferably comprising about 30% to about 40% of the total area of the first discontinuous wound-contact component (4), for allowing the first adhesive absorbent layer (3) to come into substantial contact with the wound surface.

7. A multilayer adhesive bandage for wound care according to any of claims 1 to 7, **characterized in that** the adhesive of the first adhesive absorbent layer (3) has viscosity of about 500 to about 500.000 centipoises at temperatures lower than 190°C.

8. A multilayer adhesive bandage for wound care according to claim 7, **characterized in that** the adhesive of the first adhesive absorbent layer (3) has viscosity of about 1,000 to about 30,000 centipoises at temperatures lower than 190°C.

9. A multilayer adhesive bandage for wound care according to claim 8, **characterized in that** the adhesive of the first adhesive absorbent layer (3) has viscosity of about 3,000 to about 15,000 centipoises at temperatures lower than 190°C.

10. A multilayer adhesive bandage for wound care according to any of claims 1 to 9, **characterized in that** the open area of the first discontinuous wound-contact component (4) results from a plurality of discontinuities.

11. A multilayer adhesive bandage for wound care according to claim 10, **characterized in that** the discontinuities are selected from the group of pores, tears, perforations, cracks, grooves, openings or the like, of mixtures thereof.

12. A multilayer adhesive bandage for wound care according to claim 11, **characterized in that** the area of the first discontinuous wound-contact component (4) results from a plurality of pores that have an average pore area ranging from about 0.001 to about 10 mm².

13. A multilayer adhesive bandage for wound care according to claim 12, **characterized in that** the open area of the first discontinuous wound-contact component (4) results from a plurality of pores having an average pore area ranging from about 0.01 to about 1 mm².

14. A multilayer adhesive bandage for wound care according to claim 13, **characterized in that** the open area of the first discontinuous wound-contact component (4) results from a plurality of pores having an average pore area ranging from about 0.1 to about 0.5 mm².

15. A multilayer adhesive bandage for wound care according to any of claims 2 to 14, **characterized in that** the first adhesiveness value is of 12 Newtons per square centimeter at most, and the second adhesiveness value is of 15 Newtons per square centimeter at most.

16. A multilayer adhesive bandage for wound care according to claim 15, **characterized in that** the first adhesiveness value is of 8 Newtons per square centimeters at most, and the second adhesiveness value is of 10 Newtons per square centimeter at most.

17. A multilayer adhesive bandage for wound care according to claim 16, **characterized in that** the first adhesiveness value is of 4 Newtons per square centimeter at most, and the second adhesiveness value is of 8 Newtons per square centimeters at most.

18. A multilayer adhesive bandage for wound care according to any of claims 2 to 14, **characterized in that** the first adhesiveness value is of 10 Newtons per square centimeters at most, preferably 6 Newtons per square centimeters at most, more preferably 1 Newton per square centimeter at most.

19. A multilayer adhesive bandage for wound care according to any of claims 1 to 18, **characterized in that** the first adhesive absorbent layer (3) has a first surface area and the first discontinuous wound-contact component (4) has a second surface area, and the second surface area of the first discontinuous wound-contact component (4) is, preferably about 5%, smaller than the first surface area of the first adhesive absorbent layer (3) and partially exposes the first surface area of said first adhesive absorbent layer (3).

20. A multilayer adhesive bandage for wound care according to claim 19, **characterized in that** the first adhesive absorbent layer (3) has a first surface area and the first discontinuous wound-contact component (4) has a second surface area, the second surface area of the first discontinuous wound-contact component (4) being preferably 10% smaller than the first surface area of the first adhesive absorbent layer (3) and partly exposing the first surface area of said first adhesive absorbent layer (3).

21. A multilayer adhesive bandage for wound care according to claim 20, **characterized in that** the first adhesive absorbent layer (3) has a first surface area and the first discontinuous wound-contact component (4) has a second surface area, the second surface area of the first discontinuous wound-contact component (4) being preferably at least 30% smaller than the first surface area of the first adhesive absorbent layer (3) and partly exposing the first surface area of said first adhesive absorbent layer (3).

22. A multilayer adhesive bandage for wound care according to claim 21, **characterized in that** the first adhesive absorbent layer (3) has a first surface area and the first discontinuous wound-contact component (4) has a second surface area, the second surface area of the first discontinuous wound-contact component (4) being preferably at least 50% smaller than the first surface area of the first adhesive absorbent layer (3) and partly exposing the first surface area of said first adhesive absorbent layer (3).

23. A multilayer adhesive bandage for wound care according to any of claims 19 to 22, **characterized in that** the first exposed surface of said first surface area of said first adhesive absorbent layer (3) is continuous.

24. A multilayer adhesive bandage for wound care according to any of claims 1 to 23, **characterized in that** the first discontinuous wound-contact component (4) has a contact periphery surrounding the wound, and the exposed continuous surface area of the first adhesive absorbent layer (3) is located around the periphery of said first discontinuous wound-contact component (4).

25. A multilayer adhesive bandage for wound care according to any of claims 1 to 24, **characterized in that** the first adhesive absorbent layer (3) is composed of an elastomeric base, an absorbent agent, an adhesive resin, a cohesion agent, a plasticizer and optionally a pigment.

26. A multilayer adhesive bandage for wound care according to claim 25, **characterized in that** the absorbent agent comprises a hydrocolloid.

27. A multilayer adhesive bandage for wound care according to claim 26, **characterized in that** said hydrocolloid is selected from the group of guar gums, pectin, xanthan gum, gelatins, carboxymethylcellulose, sodium alginate, calcium alginates, polysaccharides, among others, or mixtures thereof.

28. A multilayer adhesive bandage for wound care according to claim 25, **characterized in that** the absorbent agent is a superaborbent polymer, preferably a polyacrylate superabsorbent, a polyacrylate salt superabsorbent, or mixtures thereof.

29. A multilayer adhesive bandage for wound care according to any of claims 1 to 28, **characterized in that** the first adhesive absorbent layer (3) is discontinuous.

30. A multilayer adhesive bandage for wound care according to any of claims 1 to 29, **characterized in that** the first support layer (2) is a film.

31. A multilayer adhesive bandage for wound care according to claim 30, **characterized in that** the first support layer (2) is a polyolefin made film.

32. A multilayer adhesive bandage for wound care according to claim 30 or 31, **characterized in that** the first support layer (2) is a polyurethane film.

33. A multilayer adhesive bandage for wound care according to any of claims 1 to 32, **characterized in that** the first discontinuous component (4) is a net, a screen, a perforated film, a non-woven fabric, a fabric, a wax film, a powder film, wax points, a screen of a polymeric material, or a fiber grid.

34. A multilayer adhesive bandage for wound care according to any of claims 1 to 33, **characterized in that** the first discontinuous wound-contact component (4) is positioned centralized with respect to the first adhesive absorbent layer (3).

35. A multilayer adhesive bandage for wound care according to claim 1, **characterized in that** the first discontinuous wound-contact component (4) is an integral part of the first adhesive absorbent layer (3).

36. A multilayer adhesive bandage for wound care according to any of claims 1 to 35, **characterized in that** the first discontinuous wound-contact component (4) is impregnated with bactericidal agents or miscellaneous actives.

37. A multilayer adhesive bandage for wound care according to any of claims 1 to 36, **characterized in that** it reduces the re-injury of the wound when removed.

38. A multilayer adhesive bandage for wound care, in particular according to any of the preceding claims, **characterized by** comprising:
- at least one first support layer (2);
- at least one first adhesive absorbent layer (3), deposited onto the first support layer (2); and
- at least one first discontinuous wound-contact component (4), substantially free from hydrocolloids, for substantially covering a wound area, said wound area having a surface on which the first adhesive absorbent layer (3) comes into substantial contact with said wound surface through the first discontinuous wound-contact component (4).

39. A multilayer adhesive bandage for wound care, in particular according to any of the preceding claims, **characterized by** comprising at least one first support layer (2), at least one first adhesive absorbent layer (3), deposited onto the first support layer (2), at least one first discontinuous wound-contact component (4) substantially free from hydrocolloids, for substantially covering a wound area, the first discontinuous wound-contact component (4) being an integral part of said first adhesive absorbent layer (3).
